# DEMANDE DE BREVET EUROPEEN

(11) **EP 3 901 931 A2**
(43) Date de publication de la demande: **27.10.2021**
(21) Numéro de dépôt: 21170254.3
(22) Date de dépôt: 23.04.2021
(51) Int. Cl.: G08B 21/04, A61B 5/024, A61B 5/026, A61B 5/11

(54) **DISPOSITIF DE SURVEILLANCE INDIVIDUELLE DU TYPE BRACELET CONNECTE ET PROCEDE DE SURVEILLANCE D'UN UTILISATEUR CORRESPONDANT**

(30) Priorité: 23.04.2020 FR 2004050; 23.04.2020 FR 2004051
(71) Demandeur: Kerrouche, Samira, 95140 Garges-lès-Gonesse (FR); Bouyahia, Hayame, 76520 Franqueville Saint Pierre (FR)
(72) Inventeur: Kerrouche, Samira, 95140 Garges-lès-Gonesse (FR); Bouyahia, Hayame, 76520 Franqueville Saint Pierre (FR)
(74) Mandataire: Ipside

(57) **Abrégé**

L'invention concerne un dispositif (1) de surveillance individuelle, apte à venir au contact d'un poignet d'un utilisateur, comprenant un corps principal (2) muni d'un écran d'affichage (20), un bracelet (4) raccordé au corps principal (2), un module de mesure (3) destiné à venir se positionner en vis-à-vis d'une artère radiale du poignet de l'utilisateur, lorsque le dispositif est porté par l'utilisateur, un module de traitement (5), et un module de localisation (6),
ledit module de localisation (6) comprenant :
- un capteur de géolocalisation (61) ;
- un détecteur de victimes d'avalanches (62), et
des moyens (63) de communication aptes à communiquer avec une balise (9). L'invention concerne également un dispositif (1) de surveillance individuelle contre les noyades, de type bracelet connecté, apte à venir au contact d'un poignet d'un enfant, comprenant :
- au moins un premier capteur (31) apte à mesurer un premier paramètre physiologique dudit enfant ;
- au moins un deuxième capteur (32) apte à mesurer un deuxième paramètre physiologique dudit enfant ;
ledit dispositif (1) comprenant un module de traitement (5) comportant :
- des moyens (50) d'analyse du premier paramètre physiologique mesuré ;
- des moyens (51) d'analyse du deuxième paramètre physiologique mesuré, et
- des moyens de délivrance (55) d'une information indiquant la détection ou la non détection d'une anomalie en fonction de ladite analyse desdits premier et deuxième paramètres physiologiques mesurés.

## Description

### DOMAINE TECHNIQUE

La présente invention se rapporte au domaine des dispositifs de surveillance individuelle.

Plus particulièrement, l'invention concerne une montre connectée permettant d'effectuer une surveillance d'un utilisateur lorsque celui-ci évolue dans un milieu potentiellement périlleux, par exemple un randonneur, un grimpeur, un skieur, une personne effectuant de l'escalade en milieu naturel que ce soit à titre professionnel ou non. L'invention se rapporte également au domaine du repérage dans de tels milieux périlleux

Plus particulièrement, l'invention concerne également un dispositif de surveillance de personnes, notamment d'enfants, prenant la forme d'un bracelet destiné à être porté par un enfant au poignet et apte à délivrer une information caractéristique d'une anomalie en cas de danger pour l'enfant, notamment en cas de noyade. Une telle invention pourrait également être adaptée en tant que dispositif de protection individuelle contre les noyades pour d'autres catégories d'âges, par exemple pour les adolescents, les adultes, voire les séniors.

### ÉTAT DE L'ART

Les milieux relativement périlleux tels que la montagne (en été ou en hiver), la forêt, les chemins de randonnées ou sur les pistes d'escalade naturelles sont réputés pour présenter un risque important notamment du fait des conditions souvent difficiles pour y accéder par des équipes de secours.

En effet, et même si l'on sait où une personne doit être récupérée, ce qui n'est pas toujours évident du fait du peu de réseau dans un endroit donné et donc de la difficulté d'avoir une localisation précise, les équipes d'intervention sont souvent freinées par les conditions d'évolution (terrain, conditions météorologiques, ...) qui font que chaque minute gagnée peut s'avérer importante.

Par ailleurs, une personne souhaitant s'aventurer dans de tels milieux peut toujours être susceptible d'avoir un problème de santé ce qui peut vite s'avérer dangereux si elle est trop isolée.

De sorte à pouvoir anticiper ces éventuels problèmes, il existe donc un besoin de mettre en œuvre un dispositif de surveillance et de prévention en milieu périlleux qui soit sûr, fiable, et qui soit en outre suffisamment résistant pour résister, par exemple, à des chutes ou des avalanches.

Une telle solution peut être offerte par une montre connectée. De nos jours, l'utilisation des montres connectées se répand. De ce fait, ces montres sont devenues de plus en plus fonctionnelles. On connaît par exemple des bracelets connectés permettant de communiquer par audio ou vidéo, ou d'envoyer des messages. On connaît également des bracelets connectés utilisés en tant que dispositifs de surveillance, et comportant un ou plusieurs capteurs permettant de mesurer par exemple le pouls d'une personne, sa dépense de calories lors d'un exercice physique, ou des données externes telles que la température, l'humidité ambiante, ou la localisation à l'aide de données satellites.

L'avantage de telles montres connectées est qu'elles sont attachées à un poignet et sont relativement résistants, par exemple aux chutes ou aux conditions climatiques.

Toutefois, de telles montres connectées ne permettent pas, à l'heure actuelle, d'effectuer de la surveillance précise, du fait qu'en cas d'accès impossible à un réseau, une telle montre ne sera pas géolocalisée de manière exacte. En outre, de telles montres permettent d'effectuer des mesures de sorte à les afficher sur un écran mais ne font pas de traitement de ces données de sorte à alerter les secours en cas de besoin.

Il existe donc un besoin d'améliorer de telles montres connectées, et notamment de les adapter à une utilisation dans des milieux pouvant présenter un risque pour la santé de personnes.

Il existe également un besoin de fournir une telle montre connectée qui soit simple d'utilisation et peu couteuse à mettre en œuvre, de sorte que le plus grand nombre de personnes puisse en bénéficier.

D'un autre côté, les noyades accidentelles entraînent plusieurs centaines de décès chaque année en France.

Il peut s'agir d'un malaise ou d'une hydrocution, par exemple.

Chez les enfants, notamment les jeunes enfants, elles constituent la deuxième cause de décès accidentel après les accidents de la circulation. Lorsqu'elles ne sont pas suivies de décès, elles occasionnent bien souvent de lourdes séquelles, en particulier des séquelles neurologiques.

Même si des consignes de sécurité, telle que la surveillance permanente et rapprochée des jeunes enfants en tout lieu de baignade, sont souvent appliquées, et que des dispositifs de sécurité sont mis en place (barrières autour des lieux de baignade ou équipements portés par les enfants), ceci ne s'avère pas suffisant du fait qu'il est relativement facile pour un enfant d'échapper à la vigilance de ses parents et que tout dispositif de sécurité peut être contourné (les barrières peuvent être escaladées, des brassards gonflables peuvent facilement s'enlever ou se dégonfler).

Il existe donc un besoin de mettre en œuvre un dispositif de surveillance et de prévention contre la noyade qui soit sûr, fiable, et qui soit en outre difficile à retirer pour un enfant.

Une telle solution peut être offerte par un bracelet de type bracelet connecté. De nos jours, l'utilisation des bracelets connectés se répand. De ce fait, ces bracelets sont devenus de plus en plus fonctionnels. On connaît par exemple des bracelets connectés permettant de communiquer par audio ou vidéo, ou d'envoyer des messages. On connaît également des bracelets connectés utilisés en tant que dispositifs de surveillance, et comportant un ou plusieurs capteurs permettant de mesurer par exemple le pouls d'une personne, sa dépense de calories lors d'un exercice physique, ou des données externes telles que la température ou l'humidité ambiante.

L'avantage de tels bracelets connectés est qu'ils sont attachés à un poignet et sont relativement difficiles à enlever pour un enfant.

Toutefois, de tels bracelets connectés ne permettent pas, à l'heure actuelle, d'effectuer de la surveillance, notamment de la surveillance d'enfants, du fait que les mesures et analyses effectuées par de tels dispositifs ne reposent pas sur des critères qui seraient caractéristiques d'une personne en danger, notamment en danger de noyade. Ainsi, un tel dispositif de sécurité pourrait générer de fausses alertes (par exemple le pouls d'un enfant qui augmente alors que celui-ci fait simplement de l'exercice).

Il existe donc un besoin d'améliorer de tels dispositifs de sécurité, et notamment de les adapter à une utilisation dans le cadre d'une surveillance pour empêcher la noyade.

L'invention a notamment pour objectif de pallier les inconvénients de l'art antérieur.

### PRÉSENTATION DE L'INVENTION

L'invention répond à ce besoin en proposant un dispositif de surveillance individuelle de type montre connectée, apte à venir au contact d'un poignet d'un utilisateur, comprenant un corps principal muni d'un écran d'affichage, un bracelet raccordé au corps principal, un module de mesure destiné à venir se positionner en vis-à-vis d'une artère radiale du poignet de l'utilisateur, lorsque la montre est portée par l'utilisateur, un module de traitement, et un module de localisation,
ledit module de mesure comprenant :
- un premier capteur apte à mesurer le taux d'oxygène au niveau de ladite artère radiale dudit utilisateur ;
- un deuxième capteur apte à mesurer le rythme cardiaque dudit utilisateur par mesure des vibrations au niveau de ladite artère radiale ;
- un troisième capteur apte à mesurer les flux sanguins ascendant et descendant au niveau de ladite artère radiale ;
- un quatrième capteur de température cutanée dudit utilisateur,
ledit module de traitement comprenant :
- des moyens d'acquisition des données mesurées desdits premier capteur, deuxième capteur, troisième capteur et quatrième capteur ;
- des premiers moyens d'analyse du taux d'oxygène mesuré par ledit premier capteur, comparant ladite valeur du taux d'oxygène par rapport à un premier seuil prédéterminé ;
- des deuxièmes moyens d'analyse du nombre de vibrations mesuré par ledit deuxième capteur, comparant ladite valeur du rythme cardiaque par rapport à un deuxième seuil prédéterminé ;
- des troisièmes moyens d'analyse des flux sanguins ascendant et descendant mesurés par ledit troisième capteur, comparant ladite valeur du flux sanguin ascendant par rapport à un troisième seuil prédéterminé et du flux sanguin descendant par rapport à un quatrième seuil prédéterminé ;
- des quatrième moyens d'analyse de la température cutanée dudit utilisateur mesurée par ledit quatrième capteur, comparant ladite valeur de la température cutanée par rapport à un cinquième seuil prédéterminé ;
- des moyens de délivrance d'une information indiquant la détection ou la non détection d'une anomalie en fonction de ladite analyse desdits taux d'oxygène, rythme cardiaque et flux sanguins ascendant et descendant dudit utilisateur, et de ladite température cutanée dudit utilisateur,
ledit module de localisation comprenant :
- un capteur de géolocalisation ;
- un détecteur de victimes d'avalanches, et
- des moyens de communication aptes à communiquer avec une balise.

Ainsi, l'invention propose une approche nouvelle et inventive permettant de résoudre au moins en partie certains des inconvénients de l'art antérieur.

Notamment, du fait que cette montre connectée peut être portée en continu, elle permet de fournir un suivi en continu fiable et en outre un suivi adapté à une utilisation dans des milieux pouvant présenter un risque pour la santé d'un utilisateur.

Par ailleurs, du fait que ces mesures sont effectuées au niveau de l'artère radiale de l'utilisateur, cette montre connectée permet d'obtenir des données fiables et d'effectuer des analyses sur des données précises.

En outre, du fait de la pluralité de capteurs mis en œuvre ainsi que du module de traitement, les données peuvent également être recoupées ce qui amplifie cette fiabilité.

Une telle montre connectée s'avère en outre simple d'utilisation et peu couteux à mettre en œuvre.

Selon une caractéristique d'au moins un mode de réalisation de l'invention, lesdits moyens de communication comprennent des moyens de réception d'une première information délivrée par ladite balise, lorsque ladite balise se trouve à proximité de ladite montre, et des moyens de transmission d'une deuxième information vers ladite balise lorsque ladite balise se trouve à proximité de ladite montre.

De ce fait, cela permet de communiquer avec les balises et ainsi d'obtenir des informations ainsi que d'en communiquer à la balise. Une telle réalisation peut par exemple être utile pour un service de secours en cas de nécessité de retrouver une personne, ou pour une utilisation en vue d'un guidage d'un utilisateur.

Selon une caractéristique d'au moins un mode de réalisation de l'invention, ladite première information comprend au moins un élément parmi le groupe comprenant :
- l'heure de communication avec ladite balise ;
- la date de communication avec ladite balise ;
- la localisation de ladite balise ;
- un nombre de personnes ayant reçu des informations par ladite balise dans un laps de temps prédéterminé.

Selon une caractéristique d'au moins un mode de réalisation de l'invention, ladite deuxième information comprend au moins un élément parmi le groupe comprenant :
- l'heure de communication avec ladite balise ;
- la date de communication avec ladite balise ;
- un numéro d'identifiant de ladite montre connectée ;
- une information caractéristique dudit utilisateur.

Selon une caractéristique d'au moins un mode de réalisation de l'invention, la montre connectée comprend en outre un capteur apte à mesurer une pression atmosphérique et un taux d'oxygène extérieur.

De ce fait, cela permet de coupler les données recueillies avec des paramètres de l'environnement d'évolution de la montre connectée.

Selon une caractéristique d'au moins un mode de réalisation de l'invention, la montre connectée comprend en outre des moyens d'alerte et, si une information indiquant la détection d'une anomalie est délivrée par lesdits moyens de délivrance, lesdits moyens d'alerte génèrent un message d'alerte qui est transmis par le biais de moyens d'émission à au moins un contact prédéterminé.

L'invention concerne en outre un système de balisage comprenant une pluralité de balises passives géographiquement espacées d'au moins une distance et permettant d'alerter des secours ou des randonneurs de passage à basse fréquence, chacune desdites balises étant apte à communiquer avec une montre connectée selon l'un des modes de réalisation précités, lorsque ladite montre connectée se trouve à proximité de ladite balise, de sorte que ladite balise puisse délivrer une première information à ladite montre connectée et recevoir une deuxième information délivrée par ladite montre connectée.

Ainsi, cela permet, par exemple si l'utilisateur se situe dans une zone dans laquelle il n'y a pas de réseau, et que par conséquent le capteur de géolocalisation ne peut pas être actif, d'obtenir la position de l'utilisateur par le biais de balises. En outre, le système de balisage permet à la montre connectée de communiquer avec les balises et ainsi d'obtenir des informations ainsi que d'en communiquer à la balise. Une telle réalisation peut par exemple être utile pour un service de secours en cas de nécessité de retrouver une personne, ou pour une utilisation en vue d'un guidage d'un utilisateur.

L'invention concerne en outre un procédé de surveillance d'un utilisateur, ledit procédé étant apte à délivrer une information indiquant la détection ou la non détection d'une anomalie, le procédé mettant en œuvre une montre connectée apte à venir au contact d'un poignet d'un utilisateur selon l'un des modes de réalisation précités, et ledit procédé comprenant les étapes suivantes, mises en œuvre par ladite montre connectée, lorsque ledit module de mesure est positionné en vis-à-vis d'une artère radiale dudit poignet dudit utilisateur :
- mesure du taux d'oxygène dudit utilisateur, à l'aide dudit premier capteur ;
- mesure du rythme cardiaque dudit utilisateur par mesure des vibrations au niveau de ladite artère radiale, à l'aide dudit deuxième capteur ;
- mesure des flux sanguins ascendant et descendant au niveau de ladite artère radiale, à l'aide dudit troisième capteur ;
- mesure de ladite température cutanée dudit utilisateur, à l'aide dudit quatrième capteur ;
- analyse desdits taux d'oxygène, rythme cardiaque et flux sanguins ascendant et descendant, et température cutanée dudit utilisateur, de sorte à transmettre des instructions de délivrance d'une information indiquant la détection ou la non détection d'une anomalie;
- délivrance de ladite information indiquant la détection ou la non détection d'une anomalie ;
ladite étape d'analyse comprenant les étapes successives suivantes :
- comparaison du taux d'oxygène mesuré par rapport à un premier seuil prédéterminé ;
- comparaison du rythme cardiaque mesuré par rapport à un deuxième seuil prédéterminé ;
- comparaison de la valeur du flux sanguin ascendant par rapport à un troisième seuil prédéterminé et de la valeur du flux sanguin descendant par rapport à un quatrième seuil prédéterminé ;
- comparaison de valeur de la température cutanée par rapport à un cinquième seuil prédéterminé ; et
- instruction de délivrance de ladite information indiquant la détection d'une anomalie si le taux d'oxygène, et/ou le rythme cardiaque et/ou les flux sanguins ascendant et/ou descendant et/ou la température cutanée dudit utilisateur dépassent respectivement les premier, deuxième, troisième quatrième seuils et/ou cinquième seuils prédéterminés.

Selon une caractéristique d'au moins un mode de réalisation du procédé, si une information indiquant la détection d'une anomalie est délivrée par lesdits moyens de délivrance, le procédé comprend les étapes successives suivantes :
- génération d'un message d'alerte par lesdits moyens d'alerte, et
- envoi dudit message d'alerte généré audit au moins un contact prédéterminé, par le biais desdits moyens d'émission.

Selon une caractéristique d'au moins un mode de réalisation du procédé, il met en œuvre un système de balisage selon le mode de réalisation précité, et, lorsque ladite montre connectée se trouve à proximité d'une balise, le procédé comprend une étape de réception d'une première information délivrée par ladite balise et une étape de transmission d'une deuxième information à ladite balise.

L'invention concerne en outre un produit programme d'ordinateur téléchargeable depuis un réseau de communication et/ou stocké sur un support lisible par microprocesseur et/ou exécutable par un microprocesseur, caractérisé en ce qu'il comprend des instructions de code de programme pour l'exécution d'un procédé de surveillance d'un utilisateur selon l'un des modes de réalisation précités, lorsqu'il est exécuté sur un ordinateur ou un terminal mobile.

L'invention concerne en outre un médium de stockage lisible par un terminal et non transitoire, stockant un programme d'ordinateur comprenant un jeu d'instructions exécutables par un ordinateur ou un processeur pour mettre en œuvre le procédé selon l'un des modes de réalisation précités.

L'invention propose également un dispositif de surveillance individuelle d'un enfant contre les noyades, de type bracelet connecté, apte à venir au contact d'un poignet d'un enfant, comprenant un corps principal étanche, un bracelet raccordé au corps principal, et un module de mesure comprenant :
- au moins un premier capteur apte à mesurer un premier paramètre physiologique dudit enfant ;
- au moins un deuxième capteur apte à mesurer un deuxième paramètre physiologique dudit enfant ;
ledit dispositif comprenant un module de traitement comportant :
- des moyens d'analyse du premier paramètre physiologique mesuré ;
- des moyens d'analyse du deuxième paramètre physiologique mesuré, et
- des moyens de délivrance d'une information indiquant la détection ou la non détection d'une anomalie en fonction de ladite analyse desdits premier et deuxième paramètres physiologiques mesurés.

Ainsi, l'invention propose une approche nouvelle et inventive permettant de résoudre au moins en partie certains des inconvénients de l'art antérieur.

En effet, un tel bracelet connecté permet d'effectuer de la surveillance, notamment de la surveillance d'enfants, reposant sur des critères qui seraient caractéristiques d'une personne en danger, notamment en danger de noyade.

En outre, un tel dispositif de surveillance et de prévention contre la noyade s'avère, du fait de la combinaison de capteur et de l'analyse des données mesurées, qui est sûr, fiable, et qui est en outre difficile à retirer pour un enfant.

Selon une caractéristique d'au moins un mode de réalisation de l'invention :
- lesdits moyens d'analyse du premier paramètre physiologique mesuré comparent ladite valeur dudit premier paramètre physiologique par rapport à une première plage de valeur prédéterminée, et
- lesdits moyens d'analyse du deuxième paramètre physiologique mesuré comparent ladite valeur dudit deuxième paramètre physiologique par rapport à une deuxième plage de valeur prédéterminée.

Selon une caractéristique d'au moins un mode de réalisation de l'invention, le module de mesure est situé sur ledit bracelet, sur une surface du bracelet disposée en regard dudit corps principal, ledit module de mesure étant destiné à venir se positionner en vis-à-vis d'une artère radiale et/ou veine radiale du poignet de l'enfant, lorsque le bracelet est porté par l'enfant.

Selon une caractéristique d'au moins un mode de réalisation de l'invention, lesdits premier capteur et deuxième capteur sont des capteurs distincts aptes à mesurer un paramètre physiologique appartenant au groupe comprenant :
- le taux d'oxygène dans le sang ;
- le rythme cardiaque ;
- la couleur du sang ;
- la vitesse du flux sanguin ascendant ;
- la vitesse du flux sanguin descendant ;
- la température cutanée ;
- l'impédance cutanée ;
- le rythme respiratoire, ou
- une combinaison de ces paramètres.

Dans ce cas de figure :
- ledit premier capteur est un capteur mesurant le taux d'oxygène dans le sang, ladite première plage de valeur se situe entre 80% et 90%, et
- ledit deuxième capteur est un capteur mesurant le rythme cardiaque, ladite deuxième plage de valeur se situe entre 65 et 90.

Selon une caractéristique d'au moins un mode de réalisation de l'invention, le dispositif comprend au moins un troisième capteur apte à mesurer un troisième paramètre ambiant, les moyens de traitement comportant des moyens d'analyse du troisième paramètre ambiant mesuré.

Dans ce cas de figure, ledit troisième capteur est un capteur de pression.

Selon une caractéristique d'au moins un mode de réalisation de l'invention, le dispositif comprend en outre un capteur appartient au groupe comprenant :
- un capteur de géolocalisation ;
- un capteur d'humidité ;
- un capteur de température.

Selon une caractéristique d'au moins un mode de réalisation de l'invention, le module de traitement comprend également des moyens d'alerte si une information indiquant la détection d'une anomalie est délivrée par lesdits moyens de délivrance. Selon une caractéristique d'au moins un mode de réalisation de l'invention, le dispositif comprend des moyens d'activation manuelle de la surveillance.

Selon une caractéristique d'au moins un mode de réalisation de l'invention, le module de traitement comprend également des moyens de transmission à distance de ladite information indiquant la détection ou la non détection d'une anomalie délivrée par les moyens de délivrance.

Selon une caractéristique d'au moins un mode de réalisation de l'invention, le dispositif comprend une alimentation électrique.

L'invention concerne également un procédé de surveillance individuelle d'un enfant contre les noyades ledit procédé étant apte à délivrer une information indiquant la détection ou la non détection d'une anomalie, caractérisé en ce qu'il met en œuvre un dispositif de surveillance individuelle dudit enfant contre les noyades, de type bracelet connecté apte à venir au contact d'un poignet dudit enfant selon l'un des modes de réalisation de l'invention, et en ce que ledit procédé comprend les étapes suivantes, mises en œuvre par ledit dispositif de surveillance, lorsqu'il est au contact dudit poignet dudit enfant :
- mesure d'un premier paramètre physiologique dudit enfant, à l'aide dudit premier capteur ;
- mesure d'un deuxième paramètre physiologique dudit enfant, à l'aide dudit deuxième capteur ;
- analyse desdits premier et deuxième paramètres physiologiques dudit enfant, de sorte à transmettre des instructions de délivrance d'une information indiquant la détection ou la non détection d'une anomalie;
- délivrance de ladite information indiquant la détection ou la non détection d'une anomalie.

Selon une caractéristique d'au moins un mode de réalisation du procédé, l'étape d'analyse des premier et deuxième paramètres physiologiques de l'enfant comprend les sous-étapes suivantes :
- traitement du premier paramètre physiologique ;
- comparaison du premier paramètre physiologique par rapport à la première plage de valeur prédéterminée ;
- traitement du deuxième paramètre physiologique ;
- comparaison du deuxième paramètre physiologique par rapport à une deuxième plage de valeur prédéterminée, et
- instruction de délivrance de ladite information indiquant la détection d'une anomalie si lesdits premier et deuxième paramètres physiologiques ne sont pas situés respectivement dans lesdites première et deuxième plages de valeur prédéterminées.

L'invention concerne en outre un produit programme d'ordinateur téléchargeable depuis un réseau de communication et/ou stocké sur un support lisible par microprocesseur et/ou exécutable par un microprocesseur, caractérisé en ce qu'il comprend des instructions de code de programme pour l'exécution d'un procédé individuelle contre les noyades selon l'un des modes de réalisation précités, lorsqu'il est exécuté sur un ordinateur ou un terminal mobile.

L'invention concerne en outre un médium de stockage lisible par ordinateur ou un terminal mobile et non transitoire, stockant un programme d'ordinateur comprenant un jeu d'instructions exécutables par un ordinateur ou un processeur pour mettre en œuvre le procédé selon l'un des modes de réalisation précités.

L'invention concerne enfin un système de protection individuelle contre les noyades comprenant un bracelet selon l'un des modes de réalisation précités, et au moins un brassard apte à venir se mettre autour des bras d'un enfant.

### BRÈVE DESCRIPTION DES FIGURES

D'autres buts, caractéristiques et avantages de l'invention apparaîtront plus clairement à la lecture de la description suivante, donnée à titre de simple exemple illustratif, et non limitatif, en relation avec les figures, parmi lesquelles :
- Figure 1 : est un schéma illustrant en vue de côté une montre connectée selon un mode de réalisation ;
- Figure 2 : est un schéma illustrant les différents modules contenus dans la montre connectée selon le mode de réalisation de la figure 1 ;
- Figure 3 : est un schéma illustrant un procédé de surveillance d'un utilisateur selon un mode de réalisation de l'invention ;
- Figure 4 : est un schéma illustrant en détail l'étape d'analyse du procédé selon le mode de réalisation de la figure 3 ;
- Figure 5 : est un schéma illustrant un système de balisage selon un mode de réalisation de l'invention, avec une montre connectée selon le mode de réalisation de la figure 1 ;
- Figure 6 : est une vue en perspective d'un dispositif de surveillance selon un mode de réalisation de l'invention ;
- Figure 7 : est un schéma illustrant un module de mesure et un module de traitement selon un mode de réalisation de l'invention ;
- Figure 8 : est une vue en perspective éclatée d'un dispositif de surveillance selon le mode de réalisation de la figure 1 ;
- Figure 9 : est un schéma illustrant un procédé de surveillance d'un enfant selon un mode de réalisation de l'invention, et
- Figure 10 : est un schéma illustrant en détail l'étape d'analyse du procédé de suivi selon le mode de réalisation de la figure 4.
- Figure 11a : est une vue en perspective d'un dispositif de surveillance selon un deuxième mode de réalisation de l'invention ;
- Figure 11b : est une vue en perspective d'un dispositif de surveillance selon un troisième mode de réalisation de l'invention.

### DESCRIPTION DÉTAILLÉE DE MODES DE RÉALISATION

Le principe général de l'invention repose sur la mise en œuvre d'une montre connectée, apte à venir au contact d'un poignet d'un utilisateur, comprenant un corps principal muni d'un écran d'affichage, un bracelet raccordé au corps principal, un module de mesure destiné à venir se positionner en vis-à-vis d'une artère radiale du poignet de l'utilisateur, lorsque la montre est portée par l'utilisateur, un module de traitement, et un module de localisation (6), la montre pouvant indiquer la détection ou la non détection d'une anomalie en fonction de plusieurs critères qui sont mesurés au niveau de cette artère radiale.

Une telle montre permet de d'effectuer une analyse cohérente des données vitales grâce à une combinaison de capteurs, et ainsi déterminer l'état d'une personne. Elle permet également d'obtenir une position précise de l'utilisateur à un instant donné quelles que soient les conditions d'évolution. Cette détermination peut par exemple permettre d'effectuer des diagnostics en direct par des services de secours, de localiser une personne en difficulté.

Une telle montre connectée peut, par exemple, être utilisé pour effectuer une surveillance de personnes qui partent en randonnée, en montagne, au ski, ou pour toute personne devant évoluer dans des conditions pouvant être à risque.

Ce type de montre connectée s'avère simple de conception et d'utilisation.

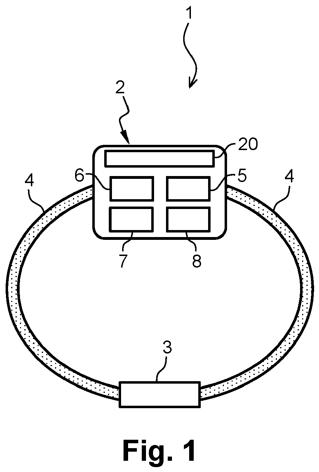

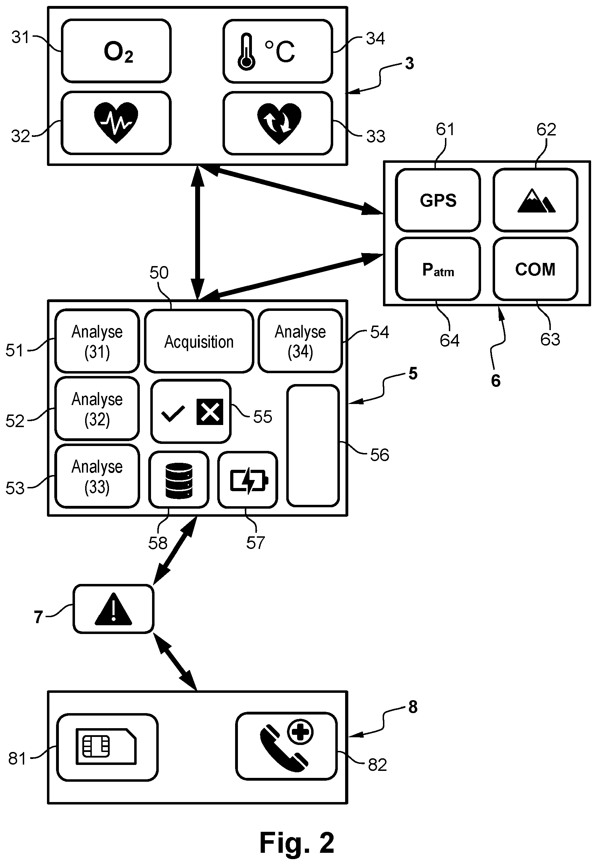

On présente maintenant, en relation avec les figures 1 et 2, un premier mode de réalisation de la montre connectée selon l'invention.

Comme illustré sur ces figures, la montre connectée 1 selon ce mode de réalisation comprend un corps principal 2 muni d'un écran d'affichage 20, un bracelet 4 raccordé au corps principal 2, un module de mesure 3, un module de traitement 5, et un module de localisation 6.

Comme décrit, cette montre est apte à venir au contact d'un poignet d'un utilisateur, de sorte que l'une des portions, plus particulièrement la portion de la montre où est logé un module de mesure 3, puisse venir se positionner en vis-à-vis d'une artère radiale du poignet de l'utilisateur, lorsque la montre est portée par cet utilisateur. Un tel corps principal 2 est, dans ce mode de réalisation, réalisé par moulage de plastique injecté de sorte à fournir un matériau étanche et résistant aux différentes conditions dans lesquelles la montre pourrait être amenée à évoluer, telles que dans des zones de températures extrêmes ou des zones de forte altitude.

On pourrait également prévoir un corps principal réalisé en un autre matériau qui permette de fournir les mêmes conditions de résistance et d'étanchéité.

Le bracelet 4 peut également être réalisé par moulage de plastique injecté ou en silicone.

Ce bracelet peut, par exemple, présenter une fermeture positionnée sur un côté, de sorte à faciliter sa mise et son retrait.

Toutefois, le bracelet pourrait également être un bracelet à fermoir ou un bracelet à verrouillage pour des questions de sécurité.

Il est à noter que la montre présentée dans ce mode de réalisation comprend une alimentation électrique. Une telle alimentation se présente ici sous la forme d'une batterie 57, ménagée au niveau des moyens de traitement 5, qui permet ainsi à cette montre d'être autonome.

Cette batterie peut, selon un mode de réalisation, être une pile rechargeable ou non. Une telle batterie pourrait, en outre, être une batterie LiPO permettant une charge rapide. Cette batterie peut être reliée à un port USB permettant de la recharger. Le rechargement pourrait également se faire par induction de sorte à éviter une entrée potentielle d'eau ou de poussière.

Le rechargement d'une telle batterie pourrait également être effectué de manière solaire, de sorte à fournir une solution écologique.

Dans ce cas, on pourrait prévoir que le rechargement solaire puisse se faire par l'intermédiaire d'un connecteur solaire associé, de sorte à pouvoir effectuer des rechargements d'urgence, par exemple en montagne ou lors de randonnées. De cette manière, cela permettrait d'éviter que la montre connectée soit à court de batterie.

Comme décrit précédemment, la montre connectée comprend un module de mesure 3. Dans ce mode de réalisation, ce module de mesure 3 comprend :
un premier capteur 31 apte à mesurer le taux d'oxygène au niveau de ladite artère radiale dudit utilisateur ;
- un deuxième capteur 32 apte à mesurer le rythme cardiaque dudit utilisateur par mesure des vibrations au niveau de ladite artère radiale ;
- un troisième capteur 33 apte à mesurer les flux sanguins ascendant et descendant au niveau de ladite artère radiale ;
- un quatrième capteur 34 de température cutanée dudit utilisateur.

Dans ce mode de réalisation, et de sorte à avoir un suivi permanent de l'utilisateur, ces mesures sont effectuées en continu.

Bien évidemment, le cas échéant, on pourrait prévoir que l'une ou l'autre de ces mesures soit effectuée de manière périodique de sorte, par exemple, à économiser la batterie de la montre connectée.

Selon un mode de réalisation non représenté, ce module de mesure pourrait également comprendre un capteur permettant de détecter une chute de l'utilisateur, par exemple un altimètre apte à détecter un changement brutal d'altitude (ici, d'altitude du poignet).

De sorte à privilégier des capteurs essentiels surveillant les fonctions vitales, il est à noter qu'un ou plusieurs des capteurs peuvent, selon un mode de réalisation, être activés ou désactivés par action de l'utilisateur, par exemple par action tactile sur l'écran d'affichage 20 ou par une combinaison de boutons.

Dans ce mode de réalisation, le module de mesure 3 est relié, par le biais d'un câble, qui est ici un câble plat, à un module de traitement 5 qui a pour but d'analyser les paramètres mesurés et de délivrer une information indiquant la détection ou la non détection d'une anomalie en fonction de ladite analyse.

Ce module de traitement 5 comporte ici :
- des moyens 50 d'acquisition des données mesurées desdits premier capteur 31, deuxième capteur 32, troisième capteur 33 et quatrième capteur 34 ;
- des premiers moyens 51 d'analyse du taux d'oxygène mesuré par ledit premier capteur 31, comparant ladite valeur du taux d'oxygène par rapport à un premier seuil prédéterminé ;
- des deuxièmes moyens 52 d'analyse du nombre de vibrations mesuré par ledit deuxième capteur 32, comparant ladite valeur du rythme cardiaque par rapport à un deuxième seuil prédéterminé ;
- des troisièmes moyens 53 d'analyse des flux sanguins ascendant et descendant mesurés par ledit troisième capteur 33, comparant ladite valeur du flux sanguin ascendant par rapport à un troisième seuil prédéterminé et du flux sanguin descendant par rapport à un quatrième seuil prédéterminé ;
- des quatrième moyens 54 d'analyse de la température cutanée dudit utilisateur mesurée par ledit quatrième capteur 34, comparant ladite valeur de la température cutanée par rapport à un cinquième seuil prédéterminé ;
- des moyens de délivrance 55 d'une information indiquant la détection ou la non détection d'une anomalie en fonction de ladite analyse desdits taux d'oxygène, rythme cardiaque et flux sanguins ascendant et descendant dudit utilisateur, et de ladite température cutanée dudit utilisateur,

Selon un mode de réalisation de l'invention, les moyens de délivrance 55 d'une information indiquant la détection ou la non détection d'une anomalie en fonction de l'analyse des taux d'oxygène, rythme cardiaque, flux sanguins ascendant et descendant, et température cutanée de l'utilisateur peuvent délivrer une information indiquant la détection d'une anomalie si au moins l'un des paramètres mesurés est caractéristique d'une anomalie.

Par exemple, en cas de baisse brutale du taux d'oxygène, visible par la mesure du taux d'oxygène puis la comparaison par rapport à un seuil prédéterminé de taux d'oxygène attendu, les moyens de délivrance peuvent délivrer une information indiquant la détection d'une anomalie.

En outre, si la comparaison de la valeur du flux sanguin ascendant par rapport à un troisième seuil prédéterminé et/ou du flux sanguin descendant par rapport à un quatrième seuil prédéterminé révèle une anomalie, cela peut être révélateur d'une pathologie cardiaque (arythmie, tachycardie, bradycardie, ou malaise cardiaque), les moyens de délivrance peuvent donc également délivrer une information indiquant la détection d'une anomalie.

De plus, en cas de baisse brutale de la température de l'utilisateur, visible par la mesure de sa température cutanée puis la comparaison par rapport à un cinquième seuil prédéterminé de température, les moyens de délivrance peuvent délivrer une information indiquant la détection d'une anomalie, par exemple une hypothermie. Selon un autre mode de réalisation, et de sorte à pouvoir réduire la possibilité d'une fausse alerte, les moyens de délivrance peuvent délivrer une information indiquant la détection d'une anomalie si au moins deux des paramètres mesurés sont caractéristiques d'une anomalie, c'est-à-dire si au moins deux des paramètres mesurés sont en dehors du seuil prédéterminé respectif.

Il est à noter que ce module de traitement 5 comprend en outre des moyens 58 de stockage des données mesurées des premier capteur 31, deuxième capteur 32, troisième capteur 33 et quatrième capteur 34.

Le fait de stocker les données mesurées des premier capteur 31, deuxième capteur 32, troisième capteur 33, et quatrième capteur 34 permet de conserver un historique des information vitales de l'utilisateur sur une période plus ou moins longue selon les modes de réalisation. De cette manière, une personne qui consulte l'historique, tel qu'un médecin ou une infirmière en cas d'intervention des secours, peut avoir une vision complète de l'historique médical de l'utilisateur de la montre connectée. Selon un mode de réalisation, la montre comprend en outre des moyens d'analyse des données mesurées et stockées sur une période prédéfinie, par exemple pour détecter d'éventuelles anomalies, telle qu'une arythmie cardiaque, une hypertension, ou une hypothermie.

Il est à noter que, selon un mode de réalisation, et pour plus d'efficacité en cas par exemple d'intervention de secours, les moyens de stockage peuvent contenir en outre au moins une information relative à l'utilisateur appartenant au groupe comprenant : l'âge de l'utilisateur, une donnée anatomique de l'utilisateur, une donnée morphologique de l'utilisateur, les antécédents médicaux de l'utilisateur, et le groupe sanguin de l'utilisateur.

Dans ce cas, et de manière à ce que la réponse apportée à une éventuelle anomalie soit la plus adaptée possible, il est préférable que :
- le premier seuil prédéterminé soit défini en fonction de ladite moins une information relative audit utilisateur, et/ou
- le deuxième seuil prédéterminé soit défini en fonction de ladite moins une information relative audit utilisateur, et/ou
- le troisième seuil prédéterminé soit défini en fonction de ladite moins une information relative audit utilisateur, et/ou
- le quatrième seuil prédéterminé soit défini en fonction de ladite moins une information relative audit utilisateur, et/ou
- le cinquième seuil prédéterminé soit défini en fonction de ladite moins une information relative audit utilisateur.

Par exemple, un seuil de rythme cardiaque ne sera pas identique que la personne soit sportive ou non, selon son âge ou sa morphologie.

De même, des antécédents médicaux peuvent être utiles pour le corps médical s'il doit effectuer une intervention opératoire. Une telle caractéristique peut donc s'avérer utile dans le cas d'intervention des secours qui peuvent avoir besoin d'accéder à certaines données d'un patient pour pouvoir intervenir rapidement. Ainsi, selon un mode de réalisation, cette ou ces informations relatives à l'utilisateur peuvent être consultées par affichage sur l'écran d'affichage 20 du corps principal 2.

Cela peut par exemple être accessible par une combinaison de touches ou par appui sur cet écran d'affichage.

Selon un mode de réalisation, un ou plusieurs des informations relatives à l'utilisateur peuvent être automatiquement affichées en cas de délivrance d'une information indiquant la détection d'une anomalie.

Selon l'invention, la montre connectée 1 comporte en outre, comme dit précédemment, un module de localisation 6. Ce module de localisation 6 comprend :
- un capteur de géolocalisation 61 ;
- un détecteur de victimes d'avalanches 62, et
- des moyens 63 de communication aptes à communiquer avec une balise 9.

Selon une technologie connue, le détecteur de victimes d'avalanches 62 est un appareil électronique émetteur-récepteur d'un signal radio particulier, destiné à localiser rapidement son porteur si celui-ci est enfoui sous une avalanche de neige, par un autre DVA manipulé à proximité par une personne portant secours. Un tel détecteur de victimes d'avalanches peut permettre d'émettre ce signal radio particulier de faible énergie même lorsque la montre n'a plus de batterie durant un laps de temps prédéterminé, par exemple durant 7 jours.

Le capteur de géolocalisation 61 permet, par exemple, à l'utilisateur de connaître sa position en lien avec une cartographie s'affichant sur l'écran 20 de la montre connectée. Cette cartographie peut également comprendre un affichage d'un trajet estimatif ainsi qu'un estimatif de temps restant avant d'atteindre un objectif de localisation saisi par l'utilisateur.

On pourrait également prévoir que la cartographie illustre, dans le cas d'une utilisation à proximité d'un domaine skiable, des indications de couleur de piste.

Une telle cartographie peut, par exemple, être téléchargeable par le biais d'une application, type application mobile, qui serait jumelée à une telle montre connectée. On pourrait également prévoir que la montre connectée intègre également une boussole. De cette manière, cela permet d'amplifier la précision des indications données quant à la position et à la direction à suivre pour un utilisateur.

On pourrait également imaginer un mode de réalisation dans lequel ce capteur de localisation est apte à partager sa localisation avec d'autres capteurs de localisation de montres connectées à proximité de sorte que la cartographie affichée sur l'écran principal puisse afficher d'autres personnes à proximité.

On pourrait encore imaginer un mode de réalisation dans lequel les localisations partagées des différentes montres puissent être synchronisées sur une application, type application mobile, de sorte à générer une cartographie d'assistance de navigation (type Waze). Une telle application mobile pourrait être utilisée pour l'escalade, le ski, la randonnée, ou tout autre activité.

En outre, et comme décrit, le module de localisation 6 comprend des moyens 63 de communication aptes à communiquer avec une balise 9.

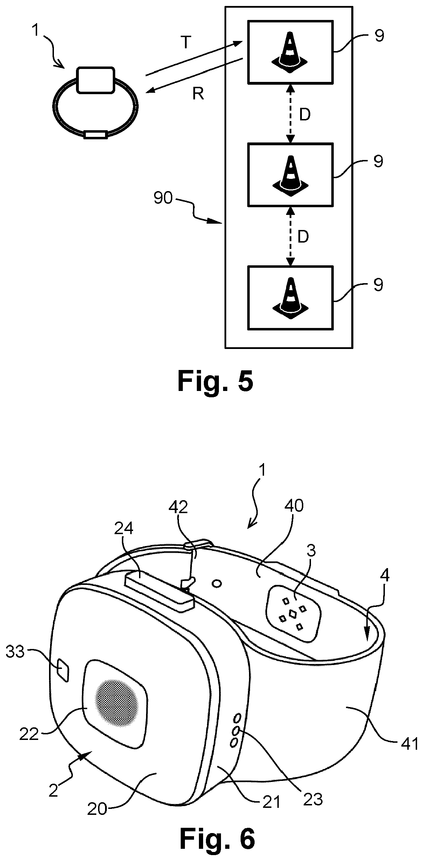

En effet, par exemple si l'utilisateur se situe dans une zone dans laquelle il n'y a pas de réseau, et que par conséquent le capteur de géolocalisation 61 ne peut pas être actif, la position de l'utilisateur ne peut pas être obtenue par ce biais-là. De ce fait, la montre connectée 1 selon l'invention est prévue pour être apte à communiquer avec un système 90 de balisage tel qu'illustré en figure 5.

Ce système 90 de balisage comprend une pluralité de balises 9 géographiquement espacées d'au moins une distance D, chacune des balises 9 étant apte à communiquer avec la montre connectée lorsque celle-ci se trouve à proximité de la balise 9, de sorte que la balise 9 à proximité puisse délivrer une première information R à la montre connectée 1 et recevoir une deuxième information T transmise par la montre connectée 1.

De ce fait, les moyens 63 de communication comprennent des moyens de réception de la première information et des moyens de transmission de la deuxième information.

La distance D peut par exemple être égale à 1 km. De ce fait, il est possible de savoir à 1km prêt la position exacte de l'utilisateur. Il est à noter qu'entre deux balises, la position de l'utilisateur peut être approximée, par exemple en fonction d'une estimation de la vitesse de l'utilisateur, du chemin emprunté.

Selon les modes de réalisation, la première information comprend au moins un élément parmi le groupe comprenant : l'heure de communication avec la balise 9, la date de communication avec la balise 9, la localisation de ladite balise 9, et un nombre de personnes ayant reçu des informations par la balise 9 dans un laps de temps prédéterminé.

En outre, la deuxième information comprend au moins un élément parmi le groupe comprenant : l'heure de communication avec la balise 9, la date de communication avec la balise 9, un numéro d'identifiant de la montre connectée 1, et une information caractéristique de l'utilisateur.

Ainsi, par un balisage à une balise donnée, un utilisateur peut par exemple connaître le nombre de personnes passées par cette balise dans un temps donné. Par exemple, on pourrait prévoir un affichage d'un nombre de personnes ayant balisé à une balise donnée dans la dernière heure. On pourrait également prévoir que la première information contienne une distance approximative à une personne ayant balisé à cette balise donnée dans la période de temps définie. Cela peut éventuellement se faire par triangulation des données.

Cela pourrait permettre que, en cas d'appartenance de l'utilisateur à un groupe, ces données puissent permettre à un membre du groupe de se situer par rapport aux autres et, par exemple, de les suivre sans les perdre.

Dans le cas d'un groupe, on pourrait par exemple prévoir que le numéro d'identifiant de la montre connecté ou que l'information caractéristique de l'utilisateur soit reconnaissable par tout le groupe (par exemple un début de numéro de série identique pour tout un groupe de montres) de sorte à faciliter l'utilisation. Une telle caractéristique peut, par exemple, être utile en vue de mettre en location de telles montres connectées, par exemple dans des bases de loisir ou dans des stations de skis en montagne.

On pourrait également prévoir que les données acquises par la balise par l'intermédiaire de la réception de la première information puisse être accessibles aux secours en cas d'intervention.

Il est à noter que, dans ce mode de réalisation, la montre connectée comprend en outre un capteur 64 apte à mesurer une pression atmosphérique et un taux d'oxygène extérieur, qui est ici placé dans le module de localisation 6. Toutefois, ce capteur pourrait également être positionné à un autre endroit de la montre connectée.

Comme illustré sur la figure 2, la montre connectée selon ce mode de réalisation comprend en outre des moyens d'alerte 7. De ce fait, si une information indiquant la détection d'une anomalie est délivrée par les moyens de délivrance 55, ces moyens d'alerte 7 génèrent un message d'alerte qui est transmis par le biais de moyens d'émission 8 par exemple à un contact prédéterminé ou à un service de secours.

On peut prévoir que la montre connectée soit apte à émettre à basse fréquence par le biais des moyens d'émission 8, même quand celle-ci est déchargée, de sorte à pouvoir être utilisée en cas d'urgence.

Ici, les moyens d'émission 8 comprennent un port 81 configuré pour recevoir une carte SIM, de préférence une carte nano SIM.

Ces moyens d'émission peuvent être accompagnés de moyens de transmission mis en œuvre sous la forme d'une antenne fonctionnant par technologie sans fil tel que Bluetooth, Wifi, 3G, 4G, ou 5G.

De cette manière, le message d'alerte peut, par exemple, être émis sous la forme d'un SMS ou d'un message vocal envoyé sur le téléphone d'une personne.

Ce message d'alerte peut également être émis sous la forme d'une notification sur un terminal mobile d'un contact prédéterminé.

Ce message d'alerte peut encore être accompagné d'une alerte envoyée à des services de secours de sorte à gagner en efficacité pour l'intervention.

Selon un mode de réalisation, le message d'alerte peut contenir des informations importantes sur l'alerte générée, par exemple la mesure qui a généré cette alerte, des informations relatives à l'utilisateur, la localisation de l'utilisateur.

Selon un mode de réalisation, le message d'alerte peut également être accompagné d'un pré-diagnostic de sorte à aiguiller plus facilement une personne vers le bon service de secours, ou pour aiguiller directement les services de secours.

Selon un mode de réalisation, le message envoyé peut également contenir un code d'accès à distance permettant aux services de secours ou au contact prédéterminé d'obtenir de nombreuses informations contenues dans la montre connectée telles que les informations de balisage par exemple.

Dans le mode de réalisation présenté, le ou les contacts prédéterminés sont stockés dans les moyens de stockage.

Selon un mode de réalisation de l'invention, ce contact prédéterminé peut également être automatiquement affiché sur l'écran d'affichage 20 en cas de délivrance d'une information indiquant la détection d'une anomalie.

On peut prévoir un mode de réalisation de l'invention dans lequel les données mesurées sont accessibles par l'utilisateur qui, par exemple par action sur l'écran d'affichage ou par combinaison de boutons, envoie des données aux secours ou au contact prédéterminé par le biais des moyens d'émission 8 de sorte à pouvoir effectuer un diagnostic à distance ou aiguiller la demande de l'utilisateur.

On peut également prévoir un mode de réalisation dans lequel les moyens d'émission sont accompagnés de moyens de réception (non représentés ici) de sorte qu'un tiers, par exemple un service de secours, puisse saisir des données à un format reconnu par la montre.

On peut également prévoir que la montre connectée comprenne des moyens d'émission d'une notification sur l'écran digital de sorte à indiquer à l'utilisateur une route conseillée ou déconseillée, une mise à jour d'informations climatiques, ...

De manière avantageuse, la montre connectée selon le mode de réalisation présenté comprend en outre des moyens d'identification uniques.

De cette manière, cela permet de sécuriser la montre connectée en limitant l'accès aux données et à son utilisation à une ou plusieurs personnes autorisées. Par exemple, dans le cas de location d'une telle montre connectée, cela permet d'empêcher leur vol.

En outre, la montre connectée peut comprendre des moyens de détection d'une mise en place de cette montre sur le poignet de l'utilisateur.

De cette manière, cela permet d'éviter des fausses alertes dans le cas d'une mesure suspecte alors que la montre ne serait pas placée sur le poignet de l'utilisateur. Selon un mode de réalisation, les moyens d'émission peuvent comprendre en outre des moyens 82 d'appel d'urgence du contact prédéterminé.

Ces moyens d'appel d'urgence peuvent être mis en œuvre sous la forme de deux boutons placés de part et d'autre du corps principal, l'utilisateur devant appuyer de manière simultanée sur les deux boutons pour effectuer cet appel d'urgence.

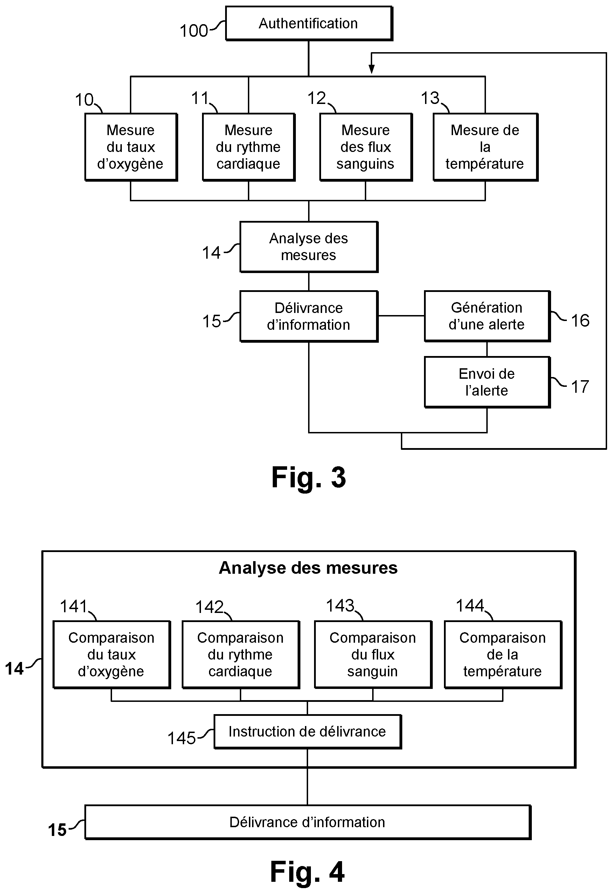

De tels moyens d'appel d'urgence peuvent également être mis en œuvre sous la forme d'un unique bouton, par exemple un bouton placé à un endroit peu accessible de la montre connectée de sorte qu'il ne puisse pas être utilisé de manière fortuite. On présente maintenant, en relation avec les figures 3 et 4, un procédé de surveillance d'un utilisateur, ce procédé étant apte à délivrer une information indiquant la détection ou la non détection d'une anomalie. Selon l'invention, ce procédé de surveillance met en œuvre une montre connectée apte à venir au contact du poignet d'un utilisateur selon l'un des modes de réalisation précités. Comme illustré, le procédé comprend les étapes suivantes, mises en œuvre par la montre connectée, lorsque le module 3 de mesure est positionné en vis-à-vis d'une artère radiale du poignet de l'utilisateur :
- mesure 10 du taux d'oxygène de l'utilisateur, à l'aide du premier capteur 31 ;
- mesure 11 du rythme cardiaque de l'utilisateur par mesure des vibrations au niveau de l'artère radiale, à l'aide du deuxième capteur 32 ;
- mesure 12 des flux sanguins ascendant et descendant au niveau de l'artère radiale, à l'aide du troisième capteur 33 ;
- mesure 13 de la température cutanée de l'utilisateur, qui se fait à l'aide du quatrième capteur 34 ;
- analyse 14 des taux d'oxygène, rythme cardiaque et flux sanguins ascendant et descendant, et température cutanée de l'utilisateur, de sorte à transmettre des instructions de délivrance d'une information indiquant la détection ou la non détection d'une anomalie ;
- délivrance 15 de l'information indiquant la détection ou la non détection d'une anomalie.

Selon l'invention, l'étape d'analyse 14 comprend les étapes successives suivantes :
- comparaison 141 du taux d'oxygène mesuré par rapport à un premier seuil prédéterminé ;
- comparaison 142 du rythme cardiaque mesuré par rapport à un deuxième seuil prédéterminé ;
- comparaison 143 de la valeur du flux sanguin ascendant par rapport à un troisième seuil prédéterminé et de la valeur du flux sanguin descendant par rapport à un quatrième seuil prédéterminé ;
- comparaison 144 de valeur de la température cutanée par rapport à un cinquième seuil prédéterminé ; et
- instruction de délivrance 145 de ladite information indiquant la détection d'une anomalie si le taux d'oxygène, et/ou le rythme cardiaque et/ou les flux sanguins ascendant et/ou descendant dudit utilisateur dépassent respectivement les premier, deuxième, troisième quatrième seuils et/ou cinquième seuils prédéterminés.

Selon un mode de réalisation de l'invention, l'étape d'instructions de délivrance 15 d'une information indiquant la détection ou la non détection d'une anomalie en fonction de l'analyse des taux d'oxygène, et/ou rythme cardiaque et/ou flux sanguins ascendant et/ou descendant et/ou température cutanée de l'utilisateur peut délivrer une information indiquant la détection d'une anomalie si au moins l'un des paramètres mesurés est caractéristique d'une anomalie.

Selon un autre mode de réalisation, et de sorte à pouvoir réduire la possibilité d'une fausse alerte, l'étape de délivrance pouvant délivrer une information indiquant la détection d'une anomalie peut être effectuée si au moins deux des paramètres mesurés sont caractéristiques d'une anomalie, c'est-à-dire si au moins deux des paramètres mesurés sont en dehors du seuil prédéterminé respectif.

Dans le mode de réalisation du procédé illustré en figure 3, si une information indiquant la détection d'une anomalie est délivrée par les moyens de délivrance 55, le procédé comprend les étapes successives suivantes :
- génération 16 d'un message d'alerte par les moyens d'alerte 7 ;
- envoi 17 du message d'alerte généré au contact prédéterminé, ou à des services de secours, par le biais des moyens d'émission.

En outre, dans le mode de réalisation illustré, le procédé comprend une étape 100 préalable d'authentification. De cette manière, cela permet de sécuriser la montre connectée en limitant l'accès aux données et à son utilisation à une ou plusieurs personnes autorisées.

L'invention concerne également un produit programme d'ordinateur téléchargeable depuis un réseau de communication et/ou stocké sur un support lisible par microprocesseur et/ou exécutable par un microprocesseur, comprenant des instructions de code de programme pour l'exécution d'un procédé de surveillance de l'utilisateur selon l'un des modes de réalisation précités, lorsqu'il est exécuté sur un ordinateur.

L'invention concerne également un médium de stockage lisible par ordinateur et non transitoire, stockant un programme d'ordinateur comprenant un jeu d'instructions exécutables par un ordinateur ou un processeur pour mettre en œuvre le procédé de surveillance de l'utilisateur selon l'un des modes de réalisation précités.

Plus particulièrement, le médium de stockage peut être inclus dans le corps principal, par exemple dans la mémoire 58 intégrée à l'endroit du module de traitement. Il pourrait également être inclus à l'endroit du module de mesure.

On pourrait également prévoir qu'une telle montre comprenne, dans cette mémoire, un échéancier de maintenance, de sorte à ce que les fonctions offertes restent optimales et que le suivi soit fiable pour un utilisateur donné.

Selon un autre aspect, le principe général de l'invention repose sur la mise en œuvre d'un dispositif de surveillance individuelle contre les noyades, de type bracelet connecté, apte à venir au contact d'un poignet d'un enfant, et pouvant indiquer la détection ou la non détection d'une anomalie en fonction d'une analyse de paramètres physiologiques mesurés de l'enfant.

Un tel bracelet peut être utilisé pour renforcer la sécurité et la surveillance de personnes, plus particulièrement de personnes vulnérables, encore plus particulièrement d'enfants, afin d'éviter les noyades.

Un tel dispositif de surveillance individuelle peut, par exemple, être utilisé en piscine privée ou municipale, dans la mer, dans des plans d'eau, ou dans n'importe quel lieu pouvant représenter un risque de noyage.

Ce type de dispositif s'avère simple de conception et d'utilisation.

On présente maintenant, en relation avec les figures 6 à 8, un premier mode de réalisation du dispositif de surveillance selon l'invention.

Comme illustré, le dispositif de surveillance 1 comprend un corps principal 2 étanche et un bracelet 4 raccordé au corps principal 2.

Le corps principal 2 présente ici une forme sensiblement carrée avec des angles arrondis et se compose d'un fond 29 surmonté de parois latérales 21, le tout étant recouvert d'une surface supérieure 20 formant couvercle.

On pourrait évidemment prévoir d'autres modes de réalisation dans lesquels ce corps principal présenterait une autre forme. Par exemple, on pourrait prévoir un corps principal rond, en losange, triangulaire, ou en forme d'étoile.

Un tel corps principal 2 est, dans ce mode de réalisation, réalisé par moulage de plastique injecté de sorte à fournir un matériau étanche et résistant aux différentes conditions dans lesquelles il pourrait être amené à évoluer, tel que l'eau de mer. On pourrait également prévoir un corps principal réalisé en un autre matériau qui permette de fournir les mêmes conditions de résistance et d'étanchéité.

Quant au bracelet 4 raccordé au corps principal 2, il présente une première portion 40 portant une surface disposée en regard du corps principal 2 et destinée venir se positionner en vis-à-vis d'une artère radiale et/ou veine radiale du poignet de l'enfant, lorsque le bracelet est porté par l'enfant, et deux deuxième portions 41 chacune relié par une extrémité au corps principal 2, l'une des deux portant en outre la première portion.

Ce bracelet 4 peut également être réalisé par moulage de plastique injecté ou en silicone.

Dans ce mode de réalisation, le bracelet 4 est formé de deux bras, l'un des bras étant constitué d'une des deux deuxièmes portions 41 relié à la première portion 40, et l'autre bras étant constitué de l'autre des deux deuxièmes portions 41. Ce bracelet présente en outre une boucle 42 portée par le bras simplement constitué de l'autre des deux deuxièmes portions 41 de sorte à venir s'insérer dans l'un des trous formés dans la première portion 40. Ainsi, un tel dispositif vient se fixer au poignet d'un enfant à la manière d'une montre à boucle classique.

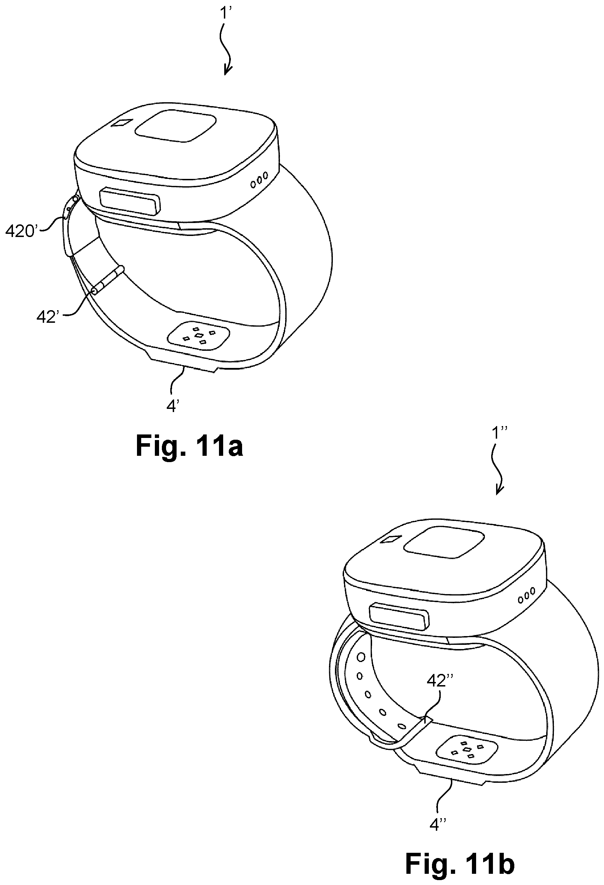

D'autres modes de réalisation du bracelet sont présentés en figures 11a et 11b.

Dans le mode de réalisation de la figure 11a, le bracelet 4' est un bracelet à fermoir, de sorte qu'un tel dispositif 4' vient se fixer au poignet d'un enfant à la manière d'une montre à fermoir classique. Deux boutons poussoirs 420' disposés de part et d'autre d'une des deux deuxièmes portions portant le fermoir permettent à un enfant d'ouvrir ce fermoir. De ce fait, cela peut permettre d'offrir une double sécurité pour éviter qu'un enfant puisse retirer le bracelet.

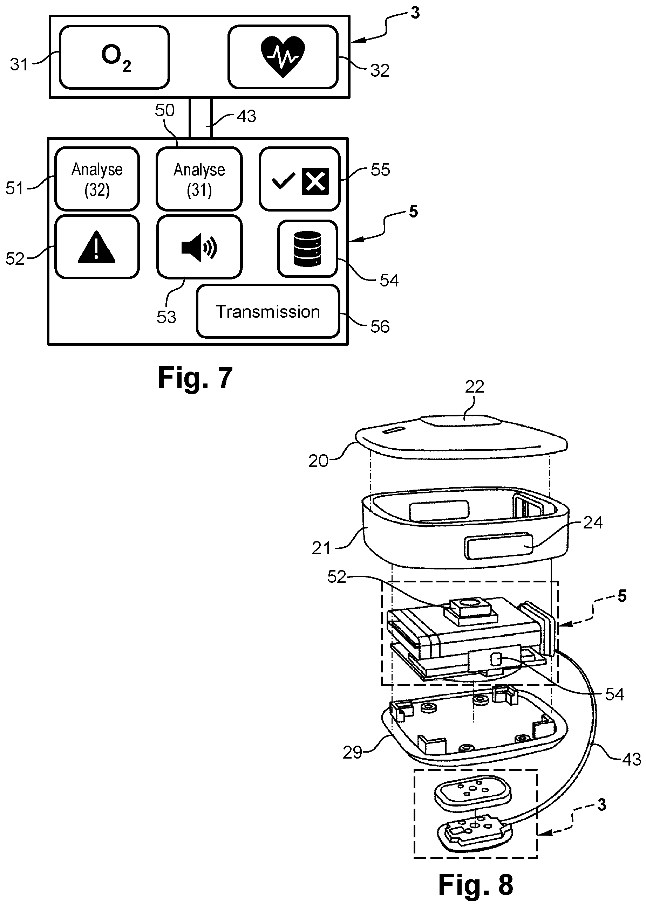

Dans le mode de réalisation de la figure 11b, le bracelet 4" est formé de deux bras, l'un des bras étant constitué d'une des deux deuxièmes portions relié à la première portion, et prolongé par une troisième portion et l'autre bras étant constitué de l'autre des deux deuxièmes portions qui vient, lorsque le bracelet est relié au poignet de l'enfant, se placer par-dessus la troisième portion et vient se fixer à elle par le biais d'une épingle en métal formée sur l'une des deux portions en vis-à-vis qui se loger dans un orifice complémentaire formé dans l'autre des deux portions en vis-à-vis. Selon l'invention, le dispositif comprend en outre un module de mesure 4. Comme plus particulièrement illustré en figure 7, le module de mesure 3 selon l'invention comprend :
- un premier capteur 31 apte à mesurer un premier paramètre physiologique de l'enfant ;
- un deuxième capteur 32 apte à mesurer un deuxième paramètre physiologique de l'enfant.

Dans le mode de réalisation illustré, les moyens 50 d'analyse du premier paramètre physiologique mesuré comparent la valeur du premier paramètre physiologique par rapport à une première plage de valeur prédéterminée, et les moyens 51 d'analyse du deuxième paramètre physiologique mesuré comparent la valeur du deuxième paramètre physiologique par rapport à une deuxième plage de valeur prédéterminée.

Plus précisément, dans le mode de réalisation illustré, le premier capteur 31 est un capteur mesurant le taux d'oxygène dans le sang, la première plage de valeur se situant entre 86% et 90%. En outre, ici, le deuxième capteur 32 est un capteur mesurant le rythme cardiaque, la deuxième plage de valeur se situant entre 65 et 90 battements par minute.

Dans un mode de réalisation, la première plage de valeur pourrait être définie par une succession de seuils franchis au sein d'une même plage de valeurs allant de 80% à 90%, par exemple un échelon à 90% puis un échelon à 88% puis un échelon à 86% et ainsi de suite, étant donné qu'en deçà de 80%, un enfant peut être considéré comme étant en hypoxie.

En effet, lorsqu'un enfant se noie, le taux d'oxygène baisse de façon régulière. Le mécanisme de noyade chez l'enfant n'est pas le même que chez l'adulte, car l'enfant se noie en inspirant de l'eau, il peut donc s'agir d'une noyade par eau ou d'une noyade sèche qui arrive à distance de la première noyade. Un tel capteur peut ainsi permettre d'évaluer à l'instant T le taux d'oxygène au moment précis où l'enfant se noie mais également à distance de la noyade pour les noyades sèches. Ici, les premier et deuxième capteurs vont marcher selon un principe d'absorbance de lumière au niveau de l'artère radiale. Le principe d'absorbance va permettre de déterminer le taux de saturation en oxygène d'un milieu, ici le sang de l'enfant. En effet, la quantité de lumière absorbée par un milieu est proportionnelle à sa concentration en une espèce chimique donnée selon la loi de Beer-Lambert. Ces capteurs qui sont donc placés sous la veine radiale sont équipés d'un émetteur-récepteur de lumière et en lien avec un processeur. L'émetteur permet l'émission d'une lumière infrarouge et d'une lumière rouge grâce à deux LED. Ces deux lumières vont traverser l'artère radiale par intermittence et vont être captées par un récepteur. Par la suite, le module de traitement constitué ici d'un nano processeur, va les quantifier. Un calcul sur la quantité de lumière absorbée va permettre de déterminer la saturation sanguine en oxygène, en isolant les variations de l'artère sur le capteur cardiaque. La saturation du sang (SpO2), s'exprime en pourcentage et va permettre d'avoir une estimation du taux. La valeur normale est située entre 90 % et 100 %. Le capteur va en outre permettre de mesurer la fréquence cardiaque en calculant le flux ascendant et descendant, par la mesure de la variation des différents flux de sang au niveau de l'artère. Les moyens d'analyse du premier paramètre sont ici composés d'un microprocesseur contenant un algorithme permettant le calcul de données et l'analyse du taux d'oxygène et les moyens d'analyse du deuxième paramètre sont composés d'un second microprocesseur calculant le taux de dioxygène pour évaluer les problèmes d'ordre cardiaque et pulmonaire dans le second bracelet.

Le module de mesure peut présenter une forme ronde ou carrée, de manière à englober toute la surface du poignet contenant l'artère radiale.

Toutefois, on pourrait prévoir d'autres modes de réalisation dans lesquels les premier et deuxième capteurs sont des capteurs distincts aptes à mesurer un paramètre physiologique appartenant au groupe comprenant :
- le taux d'oxygène dans le sang ;
- le rythme cardiaque ;
- la couleur du sang ;
- la vitesse du flux sanguin ascendant ;
- la vitesse du flux sanguin descendant ;
- la température cutanée ;
- l'impédance cutanée ;
- le rythme respiratoire, ou
- une combinaison de ces paramètres.

Par exemple, la température cutanée pourrait s'avérer utile pour détecter une éventuelle hypothermie.

Ce module de mesure 3 est, dans le mode de réalisation illustré, situé sur le bracelet 4, sur une surface de la portion 40 du bracelet qui est disposée en regard du corps principal 2. De ce fait, le module de mesure 3 est destiné à venir se positionner en vis-à-vis d'une artère radiale et/ou veine radiale du poignet de l'enfant, lorsque le bracelet est porté par l'enfant.

Une telle disposition est utile pour effectuer des mesures précises des paramètres physiologiques.

Ce module de mesure 3 est relié, par le biais d'un câble 43, qui est ici un câble plat, à un module de traitement 5 qui a pour but d'analyser les paramètres mesurés et de délivrer une information indiquant la détection ou la non détection d'une anomalie en fonction de ladite analyse.

Selon l'invention, et comme déjà décrit en partie, ce module de traitement 5 comprend :
- des moyens 50 d'analyse du premier paramètre physiologique mesuré ;
- des moyens 51 d'analyse du deuxième paramètre physiologique mesuré, et
- des moyens 55 de délivrance d'une information indiquant la détection ou la non détection d'une anomalie en fonction de ladite analyse desdits premier et deuxième paramètres physiologiques mesurés.

Dans le mode de réalisation décrit ici, le module de traitement 5 comprend également des moyens d'alerte 52, 53 si une information indiquant la détection d'une anomalie est délivrée par les moyens de délivrance 55.

Ces moyens d'alerte sont présents sous la forme d'un avertisseur sonore 52 placé en regard d'orifices 23 mis en œuvre sur une paroi latérale 21 de sorte à émettre un son d'alerte de sorte que des personnes à proximité de l'enfant puisse s'apercevoir que l'enfant est dans une situation de danger. Ces moyens d'alerte comprennent également, dans ce mode de réalisation, un avertisseur lumineux 52 émettant une lumière en cas de détection d'une anomalie, l'information pouvant donc être l'émission de lumière. Un tel avertisseur lumineux est ici mis en œuvre sous la forme d'une LED placée en regard d'une fenêtre 22 créée sur le couvercle 20 et recouverte d'un matériau au moins partiellement transparent. De cette manière, un signal lumineux, par exemple un clignotement de la LED peut alerter des personnes à proximité que l'enfant est dans une situation de danger.

Le dispositif selon le mode de réalisation décrit ici présente également des moyens 24 d'activation manuelle de la surveillance. Ces moyens sont ici mis en œuvre sous la forme de deux boutons s'étendant depuis deux côtés opposés de la paroi latérale 21 et permettant à une personne, par exemple l'un des parents de l'enfant, d'activer et/ou de désactiver le dispositif de surveillance, et également d'activer et/ou de désactiver la mise en œuvre du procédé de surveillance qui sera présenté par la suite.

Dans une mise en œuvre, pour activer ou désactiver le dispositif, une personne doit appuyer de manière simultanée sur les deux boutons.

De tels moyens d'activation manuelle de la surveillance peuvent également être mis en œuvre sous la forme d'un unique bouton, par exemple un bouton placé à un endroit peu accessible du dispositif de sorte qu'il ne puisse pas être désactivé de manière fortuite.

Un tel moyen d'activation pourrait également être mis en œuvre sous la forme d'un code à taper dans l'ordre par le biais de trois ou plus boutons, de sorte à sécuriser encore davantage l'activation et la désactivation.

Selon un autre aspect du dispositif tel que mis en œuvre dans ce mode de réalisation, le module de traitement 5 comprend également des moyens 56 de transmission à distance de l'information indiquant la détection ou la non détection d'une anomalie délivrée par les moyens de délivrance 55.

De tels moyens 56 de transmission peuvent par exemple être mis en œuvre sous la forme d'un élément apte à émettre et recevoir des données par une technologie sans fil tel que Bluetooth, Wifi, 3G, 4G, ou 5G. Un tel élément peut par exemple être associé à un port pour une carte SIM (SIM, micro Sim, nano SIM) de sorte que cette information soit émise sous la forme d'un sms à destination d'un terminal distant. Ces moyens 56 de transmission peuvent également, selon une variante, être appairés à un terminal distant de sorte à pouvoir communiquer avec une application, par exemple une application mobile, et envoyer des alertes sous forme de notifications.

Dans le mode de réalisation présenté, le dispositif 1 comprend également un troisième capteur 33 apte à mesurer un troisième paramètre ambiant, les moyens de traitement comportant des moyens d'analyse du troisième paramètre ambiant mesuré.

De cette manière, les moyens d'analyse du troisième paramètre ambiant peuvent, de manière analogue aux premier et deuxième paramètres physiologiques de l'enfant, analyser ce troisième paramètre ambiant par rapport à une troisième plage de valeur prédéterminée. En conséquence les moyens d'analyse sont aptes à délivrer une information caractéristique d'une anomalie si le premier paramètre physiologique, le deuxième paramètre physiologique, et le troisième paramètre extérieur ne sont pas situés respectivement dans les première, deuxième et troisième plages de valeur prédéterminées.

On pourrait également prévoir que l'analyse du troisième capteur soit faite par rapport à un seuil prédéterminé au lieu d'une plage de valeur prédéterminée. Dans ce mode de réalisation, le troisième capteur est un capteur de pression placé sur une puce en silicium monolithique, de sorte à fournir un capteur de pression ultra compact et fiable.

Dans le mode de réalisation illustré, le troisième capteur est un capteur de pression. De ce fait, si la pression ambiante mesurée s'avère trop importante, cela peut être, dans le cas d'un risque de noyade, une chute dans l'eau ce qui provoque ce changement brutal de pression.

Par conséquent, si les moyens d'analyse du troisième paramètre ambiant détectent que ce troisième paramètre est hors de la plage de valeur normale de pression, la pression normale correspondant par exemple à la pression de l'air libre, ils vont appuyer, en combinaison avec une anomalie si le premier paramètre physiologique et le deuxième paramètre physiologique ne sont pas non plus situés dans leur plage de valeur, à la délivrance d'une information caractéristique d'une anomalie par les moyens de délivrance.

Dans un mode de réalisation, on pourrait cependant prévoir que si seuls deux des capteurs ne sont pas situés dans leur plage de valeur, une anomalie est tout de même déclenchée.

Selon une variante, le dispositif peut en outre comprendre un capteur appartenant au groupe comprenant :
- un capteur de géolocalisation ;
- un capteur d'humidité ;
- un capteur de température.

De telles informations peuvent par exemple permettre d'obtenir des compléments, sur la localisation de l'enfant, ou une information plus précise sur la situation dans laquelle est l'enfant.

Par exemple, dans le cas d'une mise en œuvre d'un capteur de géolocalisation, une telle information pourrait être prise en compte par le module de traitement en fonction d'une localisation de l'enfant recoupée avec une carte stockée dans une mémoire indiquant des lieux potentiellement à risque.

Il est à noter que le dispositif présenté dans ce mode de réalisation comprend une alimentation électrique. Une telle alimentation se présente ici sous la forme d'une batterie, ménagée au niveau des moyens de traitement 5, qui permet ainsi à ce dispositif d'être autonome. Une telle batterie peut, par exemple, être une batterie LiPO permettant une charge rapide. Cette batterie peut être reliée à un port USB permettant de la recharger. Le rechargement pourrait également se faire par induction de sorte à éviter une entrée potentielle d'eau ou de poussière.

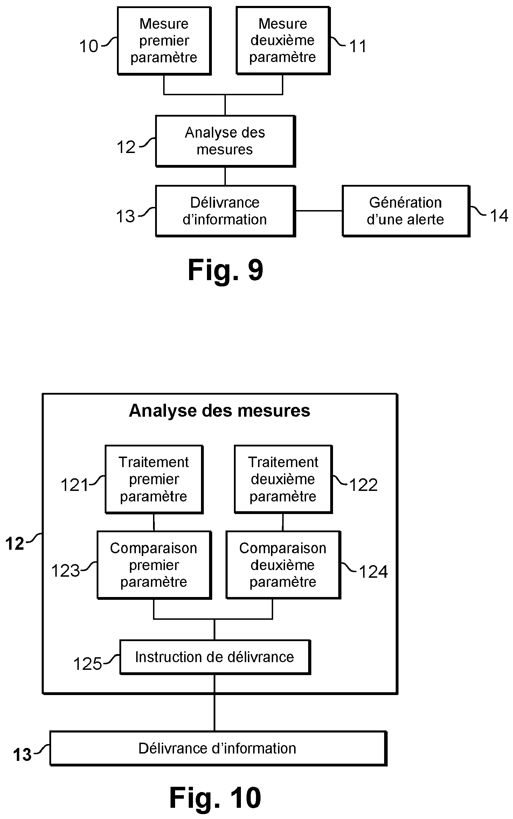

On présente par la suite, en relation avec les figures 9 et 10, un procédé de surveillance individuelle contre les noyades. Ce procédé est apte à délivrer une information indiquant la détection ou la non détection d'une anomalie, qui peut être notamment caractéristique d'un risque de noyade pour un enfant.

Selon l'invention, un tel procédé en œuvre un dispositif 1 de surveillance individuelle contre les noyades, de type bracelet connecté apte à venir au contact d'un poignet d'un enfant selon l'un des modes de réalisation précités, et comprend les étapes suivantes, mises en œuvre par ce dispositif 1 de surveillance, lorsqu'il est au contact du poignet dudit enfant (par exemple de l'enfant à surveiller) :
- mesure 10 d'un premier paramètre physiologique de l'enfant, à l'aide du premier capteur 31 ;
- mesure 11 d'un deuxième paramètre physiologique de l'enfant, à l'aide du deuxième capteur 32 ;
- analyse 12 des premier et deuxième paramètres physiologiques de l'enfant, de sorte à transmettre des instructions de délivrance d'une information indiquant la détection ou la non détection d'une anomalie ;
- délivrance 13 de l'information indiquant la détection ou la non détection d'une anomalie.

En d'autres termes, la délivrance de l'information indiquant la détection ou la non détection d'une anomalie dépend à la fois du premier et du deuxième paramètres physiologiques de l'enfant.

Dans ce mode de réalisation, l'étape d'analyse 12 des premier et deuxième paramètres physiologiques de l'enfant comprend les sous-étapes suivantes :
- traitement 121 du premier paramètre physiologique ;
- comparaison 123 du premier paramètre physiologique par rapport à la première plage de valeur prédéterminée ;
- traitement 122 du deuxième paramètre physiologique ;
- comparaison 124 du deuxième paramètre physiologique par rapport à une deuxième plage de valeur prédéterminée, etinstruction de délivrance 125 de l'information indiquant la détection d'une anomalie si les premier et deuxième paramètres physiologiques ne sont pas situés respectivement dans les première et deuxième plages de valeur prédéterminées.

Ainsi, si le premier paramètre physiologique de l'enfant, ici un enfant, se situe en dehors de la plage de valeur prédéterminée pour ce paramètre physiologique, et si le deuxième paramètre physiologique de l'enfant se situe en dehors de la plage de valeur prédéterminée pour ce paramètre physiologique, alors une instruction de délivrance d'une information indiquant la détection d'une anomalie est émise par le module de traitement.

Cette information indiquant la détection d'une anomalie peut par exemple consister en une alerte sonore émise par l'avertisseur sonore 53, ou en une alerte visuelle émise par la LED 52.

Elle peut également consister en l'émission d'une alerte sur un terminal mobile distant, par exemple un smartphone d'un des parents dans le cas d'un enfant du bracelet qui serait un enfant, à l'aide des moyens de transmission 56.

En revanche, si une information indiquant la non détection d'une anomalie est délivrée, rien ne se passe et le procédé se réitère.

Comme déjà discuté précédemment, le dispositif du premier mode de réalisation présenté comprend des moyens d'activation manuelle de la surveillance, ici sous la forme de boutons latéraux 24.

Ainsi, de tels boutons peuvent permettre d'activer et/ou de désactiver la mise en œuvre du procédé de surveillance.

Il est à noter que, selon une variante du mode de réalisation, le procédé de surveillance pourrait également comprendre une étape de désactivation au bout d'un certain laps de temps pendant lequel aucune mesure n'a pu être effectué, signe que le dispositif n'est plus relié au poignet d'un enfant. Une telle étape pourrait par exemple être utile pour économiser la batterie d'un dispositif.

Dans une variante de l'invention, on pourrait prévoir qu'un enfant soit muni d'un accessoire, tel que des brassards qui pourraient être connectés au bracelet par appairage ou par des moyens de communication sans fil.

Ces brassards peuvent être, par exemple, des brassards gonflables classiques ou des brassards présentant un matériau apte à flotter.

De tels brassards peuvent, selon un mode de réalisation, comprendre un capteur apte à envoyer des données au bracelet, par exemple au niveau des moyens de transmission 56.

Par exemple, ce capteur pourrait être un capteur de détection de mise en place sur le bras de l'enfant, une alerte étant émise dès lors qu'il est détecté que l'enfant retire ses brassards. Selon une variante, Une telle alerte pourrait présenter une condition de distance d'alerte, une alerte étant émise dès lors que l'enfant s'éloigne des brassards, par exemple à 50cm ou 1m. Une telle caractéristique pourrait permettre, par exemple, qu'un adulte soit prévenu lorsque l'enfant retire ses brassards et s'en éloigne.

De tels brassards pourraient également être reliés à une application de terminal mobile.

L'invention concerne également un produit programme d'ordinateur téléchargeable depuis un réseau de communication et/ou stocké sur un support lisible par microprocesseur et/ou exécutable par un microprocesseur, comprenant des instructions de code de programme pour l'exécution d'un procédé de surveillance individuelle contre les noyades selon l'un des modes de réalisation précités, lorsqu'il est exécuté sur un ordinateur.

L'invention concerne également un médium de stockage lisible par ordinateur et non transitoire, stockant un programme d'ordinateur comprenant un jeu d'instructions exécutables par un ordinateur ou un processeur pour mettre en œuvre le procédé de surveillance individuelle contre les noyades selon l'un des modes de réalisation précités.

Plus particulièrement, le médium de stockage peut être inclus dans le corps principal, par exemple une mémoire 54 intégrée à l'endroit du module de traitement. Il pourrait également être inclus à l'endroit du module de mesure.

## Revendications

1. Dispositif (1) de surveillance individuelle, apte à venir au contact d'un poignet d'un utilisateur, comprenant un corps principal (2) muni d'un écran d'affichage (20), un bracelet (4) raccordé au corps principal (2), un module de mesure (3) destiné à venir se positionner en vis-à-vis d'une artère radiale du poignet de l'utilisateur, lorsque le dispositif est porté par l'utilisateur, un module de traitement (5), et un module de localisation (6),
ledit module de mesure (3) comprenant :
- un premier capteur (31) apte à mesurer le taux d'oxygène au niveau de ladite artère radiale dudit utilisateur ;
- un deuxième capteur (32) apte à mesurer le rythme cardiaque dudit utilisateur par mesure des vibrations au niveau de ladite artère radiale ;
- un troisième capteur (33) apte à mesurer les flux sanguins ascendant et descendant au niveau de ladite artère radiale ;
- un quatrième capteur (34) de température cutanée dudit utilisateur,
ledit module de traitement (5) comprenant :
- des moyens (50) d'acquisition des données mesurées desdits premier capteur (31), deuxième capteur (32), troisième capteur (33) et quatrième capteur (34) ;
- des premiers moyens (51) d'analyse du taux d'oxygène mesuré par ledit premier capteur (31), comparant ladite valeur du taux d'oxygène par rapport à un premier seuil prédéterminé ;
- des deuxièmes moyens (52) d'analyse du nombre de vibrations mesuré par ledit deuxième capteur (32), comparant ladite valeur du rythme cardiaque par rapport à un deuxième seuil prédéterminé ;
- des troisièmes moyens (53) d'analyse des flux sanguins ascendant et descendant mesurés par ledit troisième capteur (33), comparant ladite valeur du flux sanguin ascendant par rapport à un troisième seuil prédéterminé et du flux sanguin descendant par rapport à un quatrième seuil prédéterminé ;
- des quatrième moyens (54) d'analyse de la température cutanée dudit utilisateur mesurée par ledit quatrième capteur (34), comparant ladite valeur de la température cutanée par rapport à un cinquième seuil prédéterminé ;
- des moyens de délivrance (55) d'une information indiquant la détection ou la non détection d'une anomalie en fonction de ladite analyse desdits taux d'oxygène, rythme cardiaque et flux sanguins ascendant et descendant dudit utilisateur, et de ladite température cutanée dudit utilisateur,
ledit module de localisation (6) comprenant :
- un capteur de géolocalisation (61) ; et
- des moyens (63) de communication aptes à communiquer avec une balise (9).

2. Dispositif (1) selon la revendication 1, **caractérisé en ce que** lesdits moyens (63) de communication comprennent des moyens de réception d'une première information délivrée par ladite balise (9), lorsque ladite balise (9) se trouve à proximité dudit dispositif (1), et des moyens de transmission d'une deuxième information vers ladite balise (9) lorsque ladite balise (9) se trouve à proximité dudit dispositif (1).

3. Dispositif (1) selon la revendication 2, **caractérisé en ce que** ladite première information comprend au moins un élément parmi le groupe comprenant :
- l'heure de communication avec ladite balise (9) ;
- la date de communication avec ladite balise (9) ;
- la localisation de ladite balise (9) ;
- un nombre de personnes ayant reçu des informations par ladite balise (9) dans un laps de temps prédéterminé.

4. Dispositif (1) selon l'une des revendications 2 ou 3, **caractérisé en ce que** ladite deuxième information comprend au moins un élément parmi le groupe comprenant :
- l'heure de communication avec ladite balise (9) ;
- la date de communication avec ladite balise (9) ;
- un numéro d'identifiant dudit dispositif (1) ;
- une information caractéristique dudit utilisateur.

5. Dispositif (1) selon l'une des revendications précédentes, **caractérisé en ce qu'**il comprend en outre un capteur (64) apte à mesurer une pression atmosphérique et un taux d'oxygène extérieur.

6. Dispositif (1) selon l'une des revendications 1 à 5, **caractérisé en ce qu'**elle comprend en outre des moyens d'alerte (7), et **en ce que**, si une information indiquant la détection d'une anomalie est délivrée par lesdits moyens de délivrance (55), lesdits moyens d'alerte (7) génèrent un message d'alerte qui est transmis par le biais de moyens d'émission (8) à au moins un contact prédéterminé.

7. Système (90) de balisage comprenant une pluralité de balises (9) passives géographiquement espacées d'au moins une distance (D), **caractérisé en ce que** chacune desdites balises (9) est apte à communiquer avec un dispositif (1) de surveillance individuelle selon l'une des revendications 1 à 6, lorsque ledit dispositif (1) se trouve à proximité de ladite balise (9), de sorte que ladite balise puisse délivrer une première information audit dispositif (1) et recevoir une deuxième information délivrée par ledit dispositif (1).

8. Procédé de surveillance d'un utilisateur, ledit procédé étant apte à délivrer une information indiquant la détection ou la non détection d'une anomalie, **caractérisé en ce qu'**il met en œuvre un dispositif (1) de surveillance individuelle apte à venir au contact d'un poignet d'un utilisateur selon l'une des revendications 1 à 6, et **en ce que** ledit procédé comprend les étapes suivantes, mises en œuvre par ledit dispositif (1), lorsque ledit module (3) de mesure est positionné en vis-à-vis d'une artère radiale dudit poignet dudit utilisateur :
- mesure (10) du taux d'oxygène dudit utilisateur, à l'aide dudit premier capteur (31) ;
- mesure (11) du rythme cardiaque dudit utilisateur par mesure des vibrations au niveau de ladite artère radiale, à l'aide dudit deuxième capteur (32) ;
- mesure (12) des flux sanguins ascendant et descendant au niveau de ladite artère radiale, à l'aide dudit troisième capteur (33) ;
- mesure (13) de ladite température cutanée dudit utilisateur, à l'aide dudit quatrième capteur (34) ;
- analyse (14) desdits taux d'oxygène, rythme cardiaque et flux sanguins ascendant et descendant, et température cutanée dudit utilisateur, de sorte à transmettre des instructions de délivrance d'une information indiquant la détection ou la non détection d'une anomalie;
- délivrance (15) de ladite information indiquant la détection ou la non détection d'une anomalie ;
ladite étape d'analyse (14) comprenant les étapes successives suivantes :
- comparaison (141) du taux d'oxygène mesuré par rapport à un premier seuil prédéterminé ;
- comparaison (142) du rythme cardiaque mesuré par rapport à un deuxième seuil prédéterminé ;comparaison (143) de la valeur du flux sanguin ascendant par rapport à un troisième seuil prédéterminé et de la valeur du flux sanguin descendant par rapport à un quatrième seuil prédéterminé ;
- comparaison (144) de valeur de la température cutanée par rapport à un cinquième seuil prédéterminé ; et
- instruction de délivrance (145) de ladite information indiquant la détection d'une anomalie si le taux d'oxygène, et/ou le rythme cardiaque et/ou les flux sanguins ascendant et/ou descendant et/ou la température cutanée dudit utilisateur dépassent respectivement les premier, deuxième, troisième quatrième seuils et/ou cinquième seuils prédéterminés.

9. Procédé selon la revendication 8, caractérisé en ce, si une information indiquant la détection d'une anomalie est délivrée par lesdits moyens de délivrance (55), le procédé comprend les étapes successives suivantes :
- génération (16) d'un message d'alerte par lesdits moyens d'alerte (7) ; et
- envoi (17) dudit message d'alerte généré audit au moins un contact prédéterminé, par le biais desdits moyens d'émission (8).

10. Procédé selon l'une des revendications 8 ou 9, **caractérisé en ce qu'**il met en œuvre un système (90) de balisage selon la revendication 9, et **en ce que**, lorsque ledit dispositif (1) se trouve à proximité d'une balise (9), le procédé comprend une étape de réception d'une première information délivrée par ladite balise (9) et une étape de transmission d'une deuxième information à ladite balise (9).

11. Dispositif (1) de surveillance individuelle d'un enfant contre les noyades, de type bracelet connecté, apte à venir au contact d'un poignet d'un enfant, comprenant un corps principal (2) étanche, un bracelet (4) raccordé au corps principal (2), et un module de mesure (3) comprenant :
- au moins un premier capteur (31) apte à mesurer un premier paramètre physiologique dudit enfant ;
- au moins un deuxième capteur (32) apte à mesurer un deuxième paramètre physiologique dudit enfant ;
ledit dispositif (1) comprenant un module de traitement (5) comportant :
- des moyens (50) d'analyse du premier paramètre physiologique mesuré ;
- des moyens (51) d'analyse du deuxième paramètre physiologique mesuré, et
- des moyens de délivrance (55) d'une information indiquant la détection ou la non détection d'une anomalie en fonction de ladite analyse desdits premier et deuxième paramètres physiologiques mesurés.
ledit module de mesure (3) est situé sur ledit bracelet (4), sur une surface (40) du bracelet disposée en regard dudit corps principal (2), ledit module de mesure (3) étant destiné à venir se positionner en vis-à-vis d'une artère radiale et/ou veine radiale du poignet de l'enfant, lorsque le bracelet est porté par l'enfant.

12. Dispositif (1) selon la revendication 11, **caractérisé en ce que** lesdits premier capteur (31) et deuxième capteur (32) sont des capteurs distincts aptes à mesurer un paramètre physiologique appartenant au groupe comprenant :
- le taux d'oxygène dans le sang ;
- le rythme cardiaque ;
- la couleur du sang ;
- la vitesse du flux sanguin ascendant ;
- la vitesse du flux sanguin descendant ;
- la température cutanée ;
- l'impédance cutanée ;
- le rythme respiratoire, ou
- une combinaison de ces paramètres.

13. Dispositif (1) selon la revendication 12, **caractérisé en ce que** :
- ledit premier capteur est un capteur mesurant le taux d'oxygène dans le sang, ladite première plage de valeur se situe entre 80% et 90%, et
- ledit deuxième capteur est un capteur mesurant le rythme cardiaque, ladite deuxième plage de valeur se situe entre 65 et 90.

14. Dispositif (1) selon l'une des revendications 11 à 13, **caractérisé en ce qu'**il comprend au moins un troisième capteur (33) apte à mesurer un troisième paramètre ambiant, les moyens de traitement comportant des moyens d'analyse du troisième paramètre ambiant mesuré.

15. Dispositif selon la revendication 14 dépendante de la revendication 13, **caractérisé en ce que** ledit troisième capteur est un capteur de pression.

16. Dispositif (1) selon l'une des revendications 11 à 15, **caractérisé en ce qu'**il comprend en outre un capteur appartient au groupe comprenant :
- un capteur de géolocalisation ;
- un capteur d'humidité ;
- un capteur de température.

17. Dispositif (1) selon l'une des revendications 11 à 16, **caractérisé en ce que** le module de traitement (5) comprend également des moyens d'alerte (52, 53) si une information indiquant la détection d'une anomalie est délivrée par lesdits moyens de délivrance (55).

18. Dispositif (1) selon l'une des revendications 11 à 17, **caractérisé en ce qu'**il comprend des moyens (24) d'activation manuelle de la surveillance.

19. Dispositif (1) selon l'une des revendications 11 à 18, **caractérisé en ce que** le module de traitement (5) comprend également des moyens (56) de transmission à distance de ladite information indiquant la détection ou la non détection d'une anomalie délivrée par lesdits moyens de délivrance (55).

20. Dispositif (1) selon l'une des revendications 11 à 19, **caractérisé en ce qu'**il comprend une alimentation électrique.

21. Procédé de surveillance individuelle d'un enfant contre les noyades ledit procédé étant apte à délivrer une information indiquant la détection ou la non détection d'une anomalie, **caractérisé en ce qu'**il met en œuvre un dispositif (1) de surveillance individuelle dudit enfant contre les noyades, de type bracelet connecté apte à venir au contact d'un poignet dudit enfant selon l'une des revendications 11 à 20, et **en ce que** ledit procédé comprend les étapes suivantes, mises en œuvre par ledit dispositif (1) de surveillance, lorsqu'il est au contact dudit poignet dudit enfant :
- mesure (10) d'un premier paramètre physiologique dudit enfant, à l'aide dudit premier capteur ;
- mesure (11) d'un deuxième paramètre physiologique dudit enfant, à l'aide dudit deuxième capteur ;
- analyse (12) desdits premier et deuxième paramètres physiologiques dudit enfant, de sorte à transmettre des instructions de délivrance d'une information indiquant la détection ou la non détection d'une anomalie;
- délivrance (13) de ladite information indiquant la détection ou la non détection d'une anomalie.

22. Procédé de surveillance d'un enfant selon la revendication 21, **caractérisé en ce que** l'étape d'analyse (12) desdits premier et deuxième paramètres physiologiques dudit enfant comprend les sous-étapes suivantes :
- traitement (121) du premier paramètre physiologique ;
- comparaison (123) du premier paramètre physiologique par rapport à la première plage de valeur prédéterminée ;
- traitement (122) du deuxième paramètre physiologique ;
- comparaison (124) du deuxième paramètre physiologique par rapport à une deuxième plage de valeur prédéterminée, et
- instruction de délivrance (125) de ladite information indiquant la détection d'une anomalie si lesdits premier et deuxième paramètres physiologiques ne sont pas situés respectivement dans lesdites première et deuxième plages de valeur prédéterminées.

23. Système de protection individuelle contre les noyades comprenant un bracelet selon l'une des revendication 11 à 20, et au moins un brassard apte à venir se mettre autour des bras d'un enfant.
